Europäisches Patentamt

European Patent Office

Office européen des brevets

(11) Publication number: **0 317 125**
**A1**

## (12) EUROPEAN PATENT APPLICATION

(21) Application number: 88310323.6

(22) Date of filing: 02.11.88

(51) Int. Cl.⁴: **C07C 19/08 , C07C 17/00**

The title of the invention has been amended (Guidelines for Examination in the EPO, A-III, 7.3).

(30) Priority: 04.11.87 GB 8725839

(43) Date of publication of application:
24.05.89 Bulletin 89/21

(84) Designated Contracting States:
AT BE CH DE ES FR GB GR IT LI LU NL SE

(71) Applicant: **IMPERIAL CHEMICAL INDUSTRIES PLC**
**Imperial Chemical House Millbank**
**London SW1P 3JF(GB)**

(72) Inventor: **Brooks, William Noel**
**19 Highbank Road**
**Kingsley Cheshire WA6 8AN(GB)**
Inventor: **Davies, Gwilym Rhys**
**Hurst View The Brow**
**Kingsley Cheshire WA6 8AN(GB)**
Inventor: **Maling, Guy Quentin**
**Church Croft Horton**
**Tilston Malpas Cheshire(GB)**

(74) Representative: **Stephenson, Kenneth**
**Imperial Chemical Industries PLC Legal**
**Department: Patents PO Box 6 Bessemer**
**Road**
**Welwyn Garden City Herts, AL7 1HD(GB)**

(54) Process for the preparation of bromofluoromethane.

(57) A method for the preparation of bromofluoromethane by reducing dibromofluoromethane in the presence of mercury.

EP 0 317 125 A1

## CHEMICAL PROCESS

This invention relates to a chemical process and more particularly to a method of making bromofluoromethane.

Bromofluoromethane is a known chemical compound which is useful as an intermediate in the production of certain pharmaceutical and agricultural chemicals. The methods that have been described for making the compound have generally given poor yields or have involved using materials which are themselves expensive such as silver fluoroacetate or not readily available such as chlorofluoromethane.

It has now been found that bromofluoromethane may be prepared in good yield by reducing dibromofluoromethane in the presence of mercury.

Thus, according to one embodiment of the invention, bromofluoromethane is prepared by treating dibromofluoromethane with an alkali metal amalgam in an active hydrogen-containing reaction medium.

The alkali metal amalgam used may be, for example, sodium amalgam which may be prepared in conventional manner by the electrolysis of an aqueous solution of sodium hydroxide or a sodium salt using a mercury cathode or by dissolving sodium in mercury. Suitable amalgams can contain from about 0.3% to about 1% by weight of sodium.

The active hydrogen-containing reaction medium is or includes a compound having one or more labile hydrogens. Suitable reaction media include alcohols, for example ethanol and propanols, and aqueous alcohols. Aprotic solvents such as acetonitrile and dimethylformamide may be used in admixture with the active hydrogen-containing compounds. Thus, the reaction medium may be a mixture of an aprotic solvent and water, a surface active agent being employed when the solvent and water are immiscible.

The reaction is exothermic and may conveniently be performed at temperatures up to the reflux temperature of the reaction mixture. The bromofluoromethane having a low boiling point (18-20°C), distils from the reaction mixture together with methyl fluoride which, having an even lower boiling point (-78°C), may easily be separated from the desired product.

In a further embodiment of the invention, bromofluoromethane is prepared electrolytically by the reduction of dibromofluoromethane at a mercury cathode.

In the electrolytic method, a solution of dibromofluoromethane in a suitable solvent, for example ethanol, together with an aqueous solution of an electrolyte is subjected to electrolysis in a cell having a mercury cathode. The electrolysis may be performed in a conventional cell, for example a divided cell, using conventional electrolytic techniques.

The invention is illustrated but not limited by the following Examples.

## EXAMPLE 1

Dibromofluoromethane (100g) is added to a flask containing sodium amalgam (5kg), propan-2-ol (140 ml) and water (20 ml), the flask being fitted with a reflux condenser and a -78°C trap. The mixture is stirred at such a rate that the exothermic reaction causes the dibromofluoromethane to reflux slowly. Bromofluoromethane distils from the reaction mixture and is trapped by the -78°C trap. The reaction is complete in about 1 hour and the temperature then falls. NMR examination ($^{19}$F) of the propan-2-ol solution at the end of the reaction indicates that no dibromofluoromethane orv bromofluoromethane is present. The methyl fluoride produced is not trapped by the -78°C trap which contains 27g of material of which 90-95% is bromofluoromethane, the remainder being dibromofluoromethane and propan-2-ol. The bromofluoromethane is separated by distillation.

## EXAMPLE 2

The procedure described in Example 1 is repeated on a smaller scale (2g dibromofluoromethane) using ethanol in place of propan-2-ol. The reaction mixture is maintained at about 30°C and the reaction is followed by means of $^{19}$F NMR. Methyl fluoride and bromofluoromethane are evolved during the reaction which is complete after 2 hours.

## EXAMPLE 3

Dibromofluoromethane is electrolysed in a glass cell consisting of two cylindrical compartments separated by a Nafion 390 cation exchange membrane. The cathode is a pool of mercury (7 cm$^2$) and the catholyte is agitated by means of a magnetic stirrer. A Drikold trap is connected to the catholyte chamber to condense evolved gases. The anolyte compartment is open to the atmosphere, a platinum strip being employed as the anode.

The cathode compartment contains dibromofluoromethane (2g) dissolved in 80/20 ethanol/water (25 ml). A 0.5 molar aqueous solution of sodium bromide is used as catholyte and anolyte. During electrolysis, a constant current (13 ma/cm²) is supplied from a potentiostat operating in galvanostatic mode. The consumption of dibromofluoromethane and the production of bromofluoromethane are followed by ¹⁹F NMR. In a typical electrolysis, 75-120% of the theoretical electrical charge is passed giving a 40% yield of bromofluoromethane. None of this material is produced when a platinum cathode is used; a lead cathode gives a negligible yield.

EXAMPLE 4

1Kg of dibromofluoromethane is added to a 5 litre flask containing 1 litre (13Kg) of sodium amalgam (amalgam strength 0.7% by weight of sodium) together with 2 litres of propan-2-ol and 300 cm³, of water. The flask is fitted with a water condenser and a -60° C cold trap.

The mixture is heated rapidly from ambient temperature to 55° C in 5 mins, the temperature then rising further with stirring to 74° C. The bromofluoromethane together with dibromofluoromethane and propan-2-ol distil over and are condensed at 0° C by an ice-cold condenser. Some of the bromofluoromethane condenses with the dibromofluoromethane/propan-2-ol mixture and the remainder is condensed in a trap at -60° C. The methyl fluoride produced is vented via the condenser.

Condensing and collecting the dibromofluoromethane and propan-2-ol reduces the methyl fluoride produced by refluxing these back into the reaction mixture with some bromofluoromethane.

978g of dibromofluoromethane in the reactor gave 305g of bromofluoromethane (53.1% yield) with 396g of dibromofluoromethane (40.5% by weight) remaining unreacted.

The bromofluoromethane is separated by distillation, the dibromofluoromethane being recycled.

EXAMPLE 5

The procedure described in Example 4 is repeated by using only the stirrer speed to raise the reaction temperature.

To the reaction flask is added 1 litre of sodium amalgam (0.6% by weight sodium) together with 2 litres of propan-2-ol, 824g of dibromofluoromethane and 300 cm³ of water. The mixture is stirred rapidly, the temperature rising over 30 mins to 75° C. The reaction is completed 50 mins after the commencement of the distillation of bromofluoromethane.

243g (50% yield) of bromofluoromethane is produced with 354g (42.9% by weight) of dibromofluoromethane remaining unreacted.

The bromofluoromethane is purified by distillation and the dibromofluoromethane is recycled.

Claims

1. A method for the preparation of bromofluoromethane which comprises reducing dibromofluoromethane in the presence of mercury.

2. A method according to claim 1 wherein dibromofluoromethane is contacted with an alkali metal amalgam in an active hydrogen-containing reaction medium.

3. A method according to claim 2 wherein the alkali metal amalgam is sodium amalgam.

4. A method according to claim 2 or claim 3 wherein the active hydrogen-containing reaction medium comprises an aqueous alcohol.

5. A method according to claim 1 wherein dibromofluoromethane is reduced electrolytically at a mercury cathode.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | EP-A-0 164 954 (IMPERIAL CHEMICAL INDUSTRIES) | | C 07 C  19/08<br>C 07 C  17/00 |
| | | | **TECHNICAL FIELDS SEARCHED (Int. Cl.4)**<br><br>C 07 C  17/00<br>C 07 C  19/00 |

The present search report has been drawn up for all claims

| Place of search | Date of completion of the search | Examiner |
|---|---|---|
| THE HAGUE | 15-03-1989 | VAN GEYT J.J.A. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)